Europäisches Patentamt

European Patent Office

Office européen des brevets

(19)

(11) Publication number: **0 069 237**

**B1**

(12) **EUROPEAN PATENT SPECIFICATION**

(45) Date of publication of patent specification: **06.11.85**

(51) Int. Cl.[4]: **A 61 M 1/32**

(21) Application number: **82104978.0**

(22) Date of filing: **07.06.82**

(54) **A device for the transfer of one or more substances between a gas and a liquid.**

(30) Priority: **07.07.81 SE 8104220**

(43) Date of publication of application:
**12.01.83 Bulletin 83/02**

(45) Publication of the grant of the patent:
**06.11.85 Bulletin 85/45**

(84) Designated Contracting States:
**AT BE CH DE FR GB IT LI LU NL**

(56) References cited:
**EP-A-0 035 266**
**US-A-4 138 464**
**US-A-4 140 635**
**US-A-4 158 693**
**US-A-4 180 896**
**US-A-4 261 951**

(73) Proprietor: **GAMBRO CARDIO AB**
**Box 10101**
**S-220 10 Lund (SE)**

(72) Inventor: **Johnsson, Bo**
**Stormgatan 39 A**
**S-261 37 Landskrona (SE)**
Inventor: **Losell, Ernst Ingvar**
**Cymbalvägen 20**
**S-245 00 Staffanstor (SE)**
Inventor: **Palmqvist, Sune Haakon**
**Nordstrands väg 19**
**S-245 00 Staffanstorp (SE)**
Inventor: **Stenberg, Kaj Ove**
**Fugavägen 53**
**S-245 00 Staffanstorp (SE)**

(74) Representative: **Boberg, Nils Gunnar Erik**
**Gambro AB Patent Department Box 10101**
**S-220 10 Lund (SE)**

Courier Press, Leamington Spa, England.

## Description

### Technical Field

The present invention relates to a device for the transfer of one or more substances between a gas and a liquid, the gas being introduced into the liquid in the form of bubbles from which the said substances are transferred to the liquid, whereafter the excess gas is removed together with any substances transferred from the liquid to the gas comprising a heat and gas exchange chamber intended to be placed vertically and provided with a heat exchanger in the form of a pipe coiled in helical manner and consisting of two parts, one of which is coiled outside the other, the two parts being separated by a first gap and forming a second gap between the outer part and the wall of the heat and gas exchange chamber, a gas inlet being arranged below the heat exchanger.

The device in accordance with the invention is intended in the first place to be used for the oxygenation of blood, that is to say as an oxygenator. It is clear, however, to those versed in the art that the construction in accordance with the invention can also be used for many purposes, that is to say substantially wherever substances from a gas are to be made to react with or to dissolve in a liquid or else if the substances are to be exchanged between gases and liquids.

### Background Art

Insofar as oxygenators are concerned, three main types can be distinguished. The oldest is probably the type where a blood film is formed in direct contact with an atmosphere containing oxygen. In a second type the oxygen is made instead to diffuse through a semipermeable membrane from an atmosphere containing oxygen on the one side of the membrane to the blood on the other side. The third type, to which belongs the device in accordance with the invention, is known under the name of bubble oxygenator. In this type the gas bubbles containing oxygen are introduced directly into the blood so as directly to act on the same. After the desired action the excess gas is removed together with carbon dioxide withdrawn from the blood.

Examples of constructions of the lastmentioned type, that is to say of bubble oxygenators, are shown for example in the following US patent specification: 3 175 555, 2 934 067, 3 545 937, 4 073 622, 3 468 631, 3 488 158, 3 615 238, 4 058 369, 3 578 411, 3 291 568, 4 033 724, 3 827 860, 3 853 479, 4 067 696, 4 065 264, 4 138 464, 4 138 288.

More particularly, the present invention constitutes a further development of the device according to EP—A—0 035 266, published after the priority date of the present application. Reference is finally made to US—A—4 158 693 describing a blood oxygenator corresponding to the preamble of claim 1 and US—A—4 261 951 describing a blood oxygenator including another kind of heat exchanger arranged in an annular heat exchange chamber.

### Disclosure of the Invention

The device in accordance with the invention is characterized in that the heat and gas exchange chamber is annular and a third gap is formed between the inner part and the inner wall of the heat and gas exchange chamber, and in that the gas inlet is in the form of a plurality of holes distributed evenly around the periphery of the chamber for the formation of bubbles which are partly broken down and/or transformed in the said gaps. By this arrangement a very compact construction of high efficiency with regard to heat transfer as well as gas exchange is achieved. The heat exchanger can be dimensioned so that it withstands the highest possible pressure which conceivably it may be subjected to. Through this arrangement the risk of leakage between the heating medium and the medium treated is reduced.

A very high safety from a point of view of leakage can be achieved if the two parts coiled in a helical manner constitute parts of one and the same pipe, which is coiled in left-hand twist and right-hand twist respectively, with the inlet and the outlet of the heat exchanger arranged at the same end of the same. This construction also facilitates installation in that the heat exchanger in a simple manner can be inserted from the top downwards into an initially open heat and gas exchange chamber, which is closed only in conjunction with the introduction of the heat exchanger.

The heat and gas exchange chamber is suitably longer than the heat exchanger itself, the latter being separated from the gas inlet by a mixing chamber. In this manner the bubbles can be formed irrespectively of the design of the heat exchanger.

In a preferred embodiment the heat and gas exchange chamber is terminated at the top by a narrow annular gap for the further breaking down of the bubbles before the liquid is introduced into a skimming chamber with a filter for the substantially complete breaking down of the bubbles. Through this arrangement the blood flow can be throttled so that an even distribution around the whole of the annular chamber is obtained.

To safeguard the size of the abovementioned gap the inner wall and/or the outer wall of the annular heat and gas exchange chamber can be provided with a spacer element which is adapted so as to hold the heat exchanger at a distance from these walls.

In order to define more closely the upper limit of the abovementioned mixing chamber the inner wall and/or the outer wall of the annular heat and gas exchange chamber may be provided with shoulders for the support of the heat exchanger.

As mentioned already, the construction in accordance with the invention is used chiefly as a so-called oxygenator, the said liquid being constituted of blood and the said gas being constituted of oxygen or an oxygen mixture from which oxygen is transferred to the blood and to which carbon dioxide is transferred from the blood. The invention is described, therefore, in the following with reference to such a construction, made up of a number of concentric vessels arranged inside one another which can easily be sealed off in relation to each other.

Brief Description of Drawings

Fig. 1 shows a longitudinal section through an oxygenator in accordance with the invention.

Figs. 2 and 3 show a side view and an end view respectively of a heat exchanger forming part of the oxygenator.

Fig. 4 shows a longitudinal section through an intermediate vessel forming part of the oxygenator in a somewhat modified design.

Fig. 5 shows an end view of the corresponding vessel seen from the open upper end of the same.

Figs. 6 and 7 finally show a section along the line VI—VI and a section perpendicular thereto through a throttling port provided at the top edge of the vessel shown in Fig. 4 and 5, respectively.

Best Mode of Carrying Out the Invention

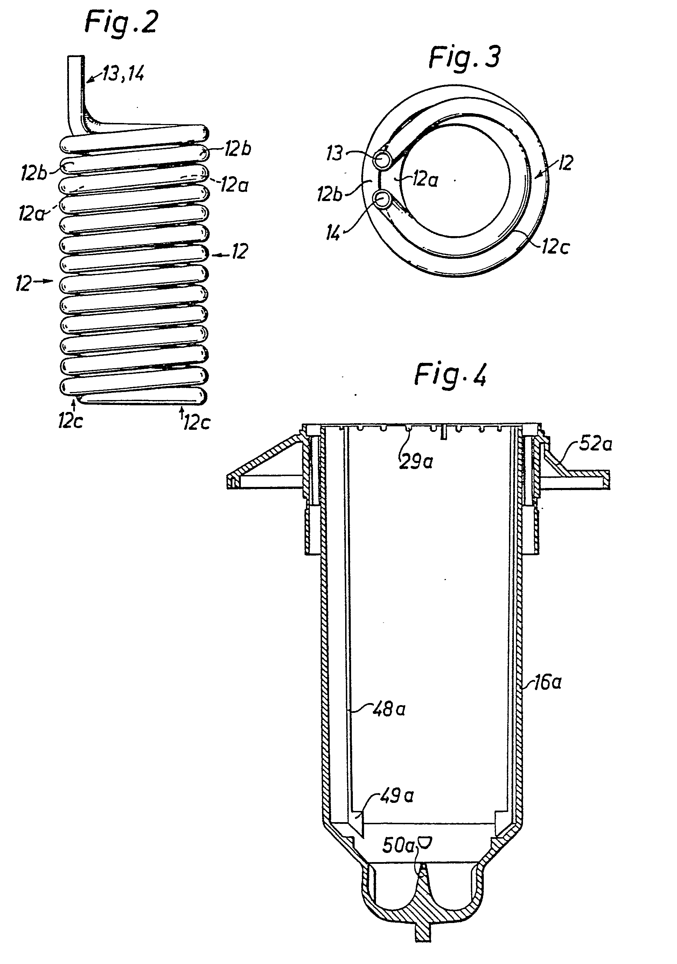

In the embodiment of the subject of the invention shown as an example venous blood is supplied from a patient to the inlet nozzle 1 arranged at the top. This nozzle together with a second inlet nozzle 2 and a sampling nozzle 3 is arranged on a branch pipe 4 which is freely rotatable in respect of the rest of the oxygenator. This branch pipe 4 is threaded onto a double-walled inlet pipe 5 arranged centrally in the oxygenator which contains an inner central liquid inlet duct 6 and an outer annular gap 7 for the gas supply. Between the inlet pipe 5 and the branch pipe 4 a packing 8 is provided. Thus, the blood is caused to flow vertically down through the duct 6 and via a smooth passage 9 out into a narrow annular gap 10. This gap subsequently passes into a widened annular vertical cylinder space 11 which contains a heat exchanger 12 and forms a heat and gas exchange chamber. The heat exchanger 12 is provided with a liquid inlet 13 and a liquid outlet 14. The construction is shown in more detail in Figs. 2 and 3. As is evident from these figures, the heat exchanger is made up of a single pipe which consists of two separate parts, namely an inner part 12a and an outer part 12b, respectively. Both these parts are coiled in helical form in left-hand twist and right-hand twist, respectively. Between the two parts there is a gap 12c. Between it and the outer and inner wall respectively of the vertical cylinder space 11, that is to say the annular heat and gas exchange chamber, the heat exchanger forms further gaps 12d and 12e, respectively.

The gap 10 and the heat and gas exchange chamber 11 formed above it are designed to be located between the centrally arranged gas and liquid inlet pipe 5 and an intermediate vessel 16 which is open at the top.

The gas, which consists of oxygen or an oxygen mixture, when the device is used as an oxygenator, is supplied via an opening 17 and an inlet chamber 18 via a sterile filter (not shown) and further ducts into the annular space 7 in the inlet pipe 5. Through a series of holes 20, which are evenly distributed along the periphery of the gap 10 at the upper end of the latter, the gas is then pressed out into the blood just where the gap 10 passes over into the widened cylinder space 11. Through this arrangement a very effective mixture of blood and gas is achieved. The bubble formation is facilitated, in that the lower part of the space 11 is designed as a mixing chamber which is clear of the heat exchanger. The mixing process is improved further in the following heat exchanger 12 with its gaps 12c, 12d and 12e.

The mixture of gas and liquid thus flows vertically upwards in the said gaps. In the upper end of the heat exchanger the gas and liquid mixture flows through an annular throttling gap 29. This gap may thus be arranged in form of a spillway between the top edge of the vessel 16 and a lid 30 provided above the same. This lid is designed so as to be integral with the double-walled inlet pipe 5 and is connected in turn via seals 31 to an outer lid 32 comprising, among other things, the gas inlet chamber 18 and the sterile filter (not shown).

Preferably, however, it was found appropriate to replace the gap 29 by the throttling ports 29a shown in Figs. 4—7 which will be described in more detail in connection with these figures.

From the spillway 29 or the throttling ports 29a the gas mixture flows down through a gap 33 between the intermediate vessel 16 and a filter 34. The blood flows out through the filter 34 into a blood collecting space 35, whilst the excess gas flows out instead through an outlet 36.

The sampling nozzle 3 is intended for the sampling of venous blood. Via the sampling nozzle 37 and a pipe (not shown) it is possible instead to take samples of arterial blood owing to this pipe terminating down in the blood collecting space 35.

The intermediate vessel 16 with the inlet pipe 5 arranged therein and the filter 34 arranged outside the same, are enclosed in an outer casing 39 comprising a bottom outlet 40 for the separated fluid phase. The lower part of the bottom of this outer casing 39 is designed as an annular gap 41 with a base 42 sloping evenly towards the bottom outlet 40.

It can be said that the construction as a whole is composed of a number of concentric vessels inserted into each other. The outermost of these is the outer casing 39. Into this is inserted a "vessel" formed of the filter 34 which is limited at the bottom by a base ring 43 and at the top by a fixing ring 44. With the help of the ring 44 the filter is fixed to the outside of the vessel 16 described above as the intermediate vessel. Into the latter is then inserted the inlet pipe 5 designed as a double-walled vessel.

The base ring 43 and the fixing ring 44 are designed so that the filter 34 forms an angle against the outer wall of the vessel 16. Through this arrangement a conical space 46 is formed for the collection of the gas and liquid mixture before the same is passed through the filter 34. In this way the blood is made in the first place to pass through the lower part of the filter and to make use only at higher flow also of the upper parts of the filter 34.

Numeral 45 designates an inlet nozzle intended for the supply of medicine, heparin or other additions to the mixture of gas and blood before the same passes the filter 34.

With regard to the holes 20 it may be said that in a preferred embodiment they are constituted of a number of approximately 100 with an accurately defined diameter. This can be made to be practically exactly 25 μ, for example, with the help of a laser technique. With a length of the holes of approximately 3 mm it was found that air will flow through all the holes, even at a gas flow as low as 0.75 litre per minute.

In this preferred embodiment the gap 10 is approximately 3 mm, which provides a good mixture at a standard blood flow at between 2 and 6 litre per minute. However, the device may also be used at higher as well as at lower flow.

Numeral 47 finally designates a number of caps for the closing of different openings of the oxygenator.

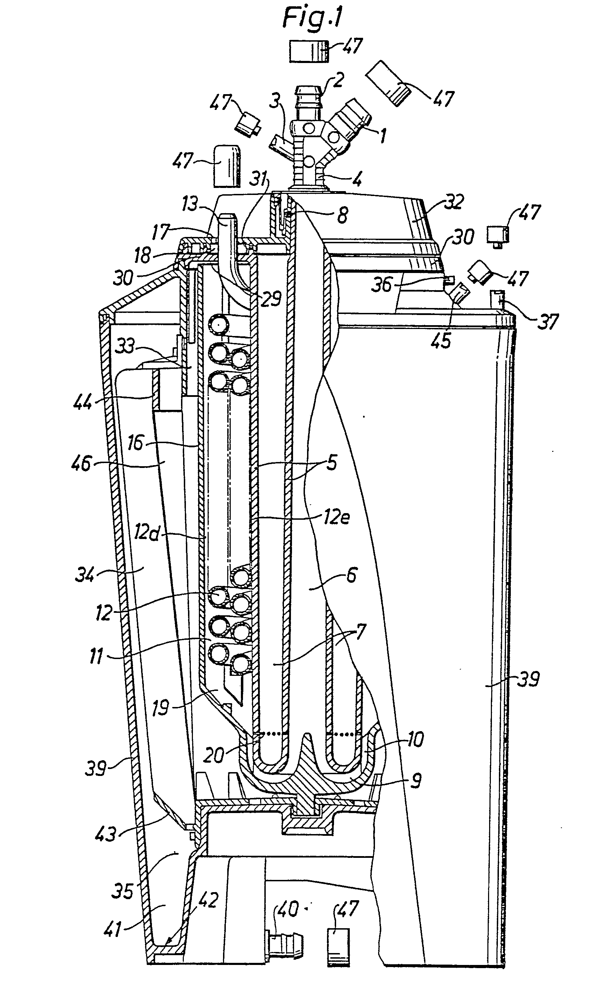

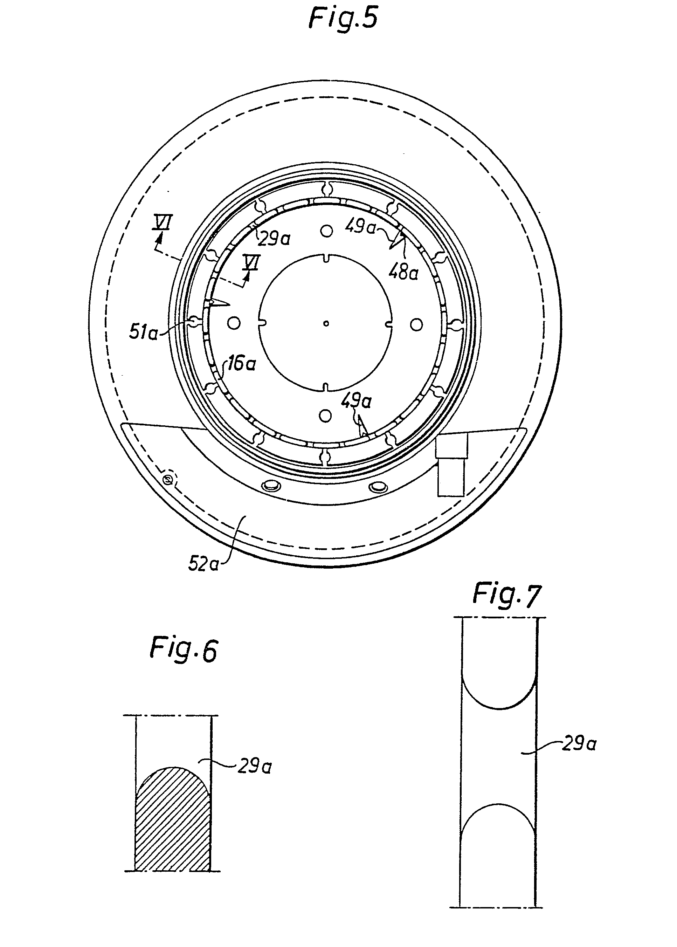

The vessel shown in Figs. 4 and 5 corresponds in principle to the vessel 16 in Fig. 1. It has been somewhat modified, however, and for this reason the same reference designations have been used, but with the addition of a small a. The vessel is thus designated 16a and the throttling ports, which replace the gap 29, are designated 29a. Numeral 48a designates three ribs which together define the size of the gap, corresponding to the gap 12d formed between the heat exchanger 12 and the vessel 16a. Three shoulders 49a define the lower limit of the heat exchanger or the upper limit of the mixing chamber corresponding to the chamber 19 in Fig. 1. Numeral 50a designates a point which is designed so as to ensure that the blood is deflected smoothly from a flow directed vertically downwards to one directed upwards. The intermediate stage 51a is provided with thickened portions so as to facilitate the outflow of the plastic when the vessel 16a and the lid 52a attached are manufactured by injection moulding.

As is evident from figures 6 and 7 the ports 29a are of a double-curved shape. This shape is very important, as it ensures that the blood is handled gently in spite of these ports being subjected to a throttling.

Naturally the invention is not limited to the embodiment described above, but can be varied within the scope of the subsequent claims. The individual shape of the components present, for example, may be varied within wide limits. See also EP—A—0 069 236 submitted at the same time and the abovementioned EP—A—0 035 266.

## Claims

1. A device for the transfer of one or more substances between a gas and a liquid, the gas being introduced into the liquid in the form of bubbles from which the said substances are transferred to the liquid, whereafter the excess gas is removed together with any substances transferred from the liquid to the gas, comprising a heat and gas exchange chamber (11) intended to be placed vertically and provided with a heat exchanger (12) in the form of a pipe coiled in helical manner and consisting of two parts (12a, 12b), one of which (12b) is coiled outside the other (12a), the two parts (12a, 12b) being separated by a first gap (12c) and forming a second gap (12d) between the outer part and the wall of the heat and gas exchange chamber, a gas inlet (20) being arranged below the heat exchanger (12), characterized in that the heat and gas exchange chamber is annular and a third gap (12e) is formed between the inner part (12a) and the inner wall of the heat and gas exchange chamber and in that the gas inlet is in the form of a plurality of holes distributed evenly around the periphery of the chamber for the formation of bubbles which are partly broken down and/or transformed in the said gaps (12c, 12d, 12e).

2. A device in accordance with claim 1, characterized in that the two parts (12a, 12b) coiled in helical manner constitute parts of one and the same pipe, which is coiled in left-hand twist and right-hand twist, respectively, with the inlet and the outlet (13, 14) of the heat exchanger arranged at the same end of the same.

3. A device in accordance with any one of the preceding claims, characterized in that the heat and gas exchange chamber (11) is longer than the heat exchanger (12) itself, the latter being separated from the gas inlet (20) by a mixing chamber (19).

4. A device in accordance with claim 1, characterized in that the heat and gas exchange chamber (11) is terminated at the top by a narrow annular gap (29) for the further breaking down of the bubbles before the liquid is introduced into a skimming chamber (33, 46) with a filter (34) for the substantially complete breaking down of the bubbles.

5. A device in accordance with any one of the preceding claims, characterized by spacer elements (48a) arranged on the inner wall and/or outer wall of the annular heat and gas exchange chamber (11) which are adapted so as to hold the heat exchanger (12) at a distance from these walls to form the gaps between these walls and the heat exchanger.

6. A device in accordance with any one of the preceding claims, characterized by shoulders (49a) arranged on the inner wall and/or outer wall of the annular heat and gas exchange chamber (11) for the support of the heat exchanger.

## Revendications

1. Dispositif pour effectuer le transfert d'une ou plusieurs substances entre un gaz et un liquide, le gaz étant introduit dans le liquide sous forme de bulles à partir desquelles lesdites substances sont transférées au liquide, après quoi l'excès de gaz est éliminé avec toutes les substances transférées du liquide au gaz, ledit dispositif comprenant une chambre d'échange thermique et gazeux (1) conçue pour être disposée verticalement et munie d'un échangeur thermique (12) constitué par un tuyau enroulé de façon hélicoïdale et consistant en deux parties (12a, 12b) dont l'une (12b) est enroulée à l'extérieur de l'autre (12a), les deux parties (12a, 12b) étant séparées par un premier intervalle (12c) et formant un deuxième intervalle (12d) entre la partie extérieure et la paroi de la chambre d'échange thermique et gazeux, une entrée pour le gaz (20) étant disposée sous l'échangeur thermique (12), caractérisé en ce que la chambre d'échange thermique et gazeux est annulaire et un troisième intervalle (12e) est formé entre la partie intérieure (12a) et la paroi intérieure de la chambre d'échange thermique et gazeux, et en ce que l'entrée pour le gaz est constituée par une pluralité de trous répartis régulièrement autour de la périphérie de la chambre pour la formation de bulles qui sont partiellement fracturées et/ou transformées dans lesdits intervalles (12c, 12d, 12e).

2. Dispositif suivant la revendication 1, caractérisé en ce que les deux parties (12a, 12b) enroulées de façon hélicoïdale constituent les parties d'un seul et même conduit qui est enroulé en une torsion vers la gauche et une torsion vers la droite, respectivement, avec l'orifice d'entrée et l'orifice de sortie (13, 14) de l'échangeur thermique disposés à la même extrémité dudit échangeur.

3. Dispositif suivant l'une quelconque des revendications précédentes, caractérisé en ce que la chambre d'échange thermique et gazeux (11) est plus longue que l'échangeur thermique (12) proprement dit, ce dernier étant séparé de l'entrée de gaz (20) par une chambre de mélange (19).

4. Dispositif suivant la revendication 1, caractérisé en ce que la chambre d'échange thermique et gazeux (11) se termine à son extrémité supérieure par un étroit intervalle annulaire (29) pour un fractionnement supplémentaire de bulles avant introduction du liquide dans une chambre de séparation (33, 46) avec un filtre (34) pour le fractionnement sensiblement complet des bulles.

5. Dispositif suivant l'une quelconque des revendications précédentes, caractérisé en ce que des éléments d'espacement (48a) sont disposés sur la paroi intérieure et/ou sur la paroi extérieure de la chambre annulaire d'échange thermique et gazeux (11) afin de maintenir l'échangeur thermique (12) à distance de ces parois pour former les intervalles entre ces parois et l'échangeur thermique.

6. Dispositif suivant l'une quelconque des revendications précédentes, caractérisé par des épaulements (49a) disposés sur la paroi intérieure et/ou sur la paroi extérieure de la chambre annulaire d'échange thermique et gazeux (11) pour le support de l'échangeur thermique.

**Patentansprüche**

1. Vorrichtung zur Übertragung einer oder mehrerer Substanzen zwischen einem Gas und einer Flüssigkeit, wobei das Gas in die Flüssigkeit in Form von Blasen eingeführt wird, aus welcher die Substanzen zu der Flüssigkeit übertragen werden, wonach das überschüssige Gas zusammen mit irgendwelchen aus der Flüssigkeit zu dem Gas übertragenen Substanzen entfernt wird, mit einer Wärme- und Gasaustauscherkammer (11), die vertikal angeordnet werden soll und mit einem Wärmetauscher (12) in Form einer Leitung vorgesehen ist, die schraubenförmig gewickelt ist und aus zwei Teilen (12a, 12b) besteht, deren einer (12b) außerhalb des anderen (12a) gewickelt ist, wobei die zwei Teile (12a, 12b) durch einen ersten Spalt (12c) getrennt sind und einen zweiten Spalt (12d) zwischen dem äußeren Teil und der Wärme- und Gasaustauscherkammer bilden und ein Gaseiblaß (20) unter dem Wärmetauscher (12) angeordnet ist, dadurch gekennzeichnet, daß die Wärme- und Gasaustauscherkammer ringförmig ist und ein dritter Spalt (12e) zwischen dem inneren Teil (12a) und der Innenwand der Wärme- und Gasaustauscherkammer gebildet ist und daß der Gaseinlaß in Form einer Mehrzahl von Löchern ist, die gleichmäßig um den Umfang der Kammer für die Bildung von Blasen verteilt sind, welche teilweise aufgebrochen und/oder in die Spalte (12c, 12d, 12e) übertragen werden.

2. Vorrichtung nach Anspruch 1, dadurch gekennzeichnet, daß die zwei Teile (12a, 12b), die schraubenförmig gewickelt sind, Teile ein und derselben Leitung bilden, die mit Linksdrehung bzw. Rechtsdrehung gewickelt sind, wobei der Einlaß und der Auslaß (13, 14) des Wärmetauschers an demselben Ende desselben angeordnet sind.

3. Vorrichtung nach einem der vorhergehenden Ansprüche, dadurch gekennzeichnet, daß die Wärme- und Gasaustauscherkammer (11) länger ist als der Wärmetauscher (12) selbst, wobei letzterer von dem Gaseinlaß (20) durch eine Mischkammer (19) getrennt ist.

4. Vorrichtung nach Anspruch 1, dadurch gekennzeichnet, daß die Wärme- und Gasaustauscherkammer (11) oben durch einen Ringspalt (29) für das weitere Aufbrechen der Blasen begrenzt ist, bevor die Flüssigkeit in eine Entschäumungskammer (33, 46) mit einem Filter (34) für das nachfolgende vollständige Aufbrechen der Blasen eingeführt ist.

5. Vorrichtung nach einem der vorhergehenden Ansprüche, gekennzeichnet durch Abstandselemente (48a), die auf der Innenwand und/oder Außenwand der ringförmigen Wärme- und Gasaustauscherkammer (11) angeordnet sind, die geeignet derart ausgestaltet sind, daß sie den Wärmetauscher (12) in einem Abstand von diesen Wänden halten, um die Spalte zwischen diesen Wänden und dem Wärmetauscher zu bilden.

6. Vorrichtung nach einem der vorhergehenden Ansprüche, gekennzeichnet durch Schultern (49a), die auf der Innenwand und/oder Außenwand der ringförmigen Wärme- und Gasaustauscherkammer (11) für die Abstützung des Wärmetauschers angeordnet sind.

Fig.1

## Fig.2

13,14

12b

12b

12a

12a

12

12

12b

12a

12c 12c

## Fig.3

13

12b

14

12

12a

12c

## Fig.4

29a

52a

16a

48a

49a

50a

## Fig.5

## Fig.6

## Fig.7